# EUROPEAN PATENT SPECIFICATION

(11) **EP 2 990 744 B1**
(45) Date of publication and mention of the grant of the patent: **31.05.2017**
(21) Application number: 14787458.0
(22) Date of filing: 03.04.2014
(51) Int. Cl.: F25D 3/00, A61K 35/12, A61K 35/14, A61K 35/56

(54) **FREEZING DEVICE**
TIEFKÜHLVORRICHTUNG
DISPOSITIF DE CONGÉLATION

(30) Priority: 23.04.2013 JP 2013090634
(43) Date of publication of application: 02.03.2016
(73) Proprietor: Technican Co., Ltd., Kanagawa 224-0037 (JP)
(72) Inventor: YAMADA, Yoshio, Yokohama-shi Kanagawa 224-0037 (JP)
(74) Representative: Colombo, Stefano Paolo
(86) International application number: PCT/JP2014/001945
(87) International publication number: WO 2014/174777

(56) References cited:
- EP-A1- 1 552 758
- JP-A- S6 017 674
- JP-A- S6 434 226
- JP-A- H06 174 353
- JP-A- 2002 090 019
- JP-A- 2005 192 533
- JP-U- 3 151 617
- JP-U- S6 369 976
- JP-U- S49 111 864

## Description

### TECHNICAL FIELD

The present invention relates to a freezing apparatus that freezes an object to be frozen using a freezing medium.

### BACKGROUND ART

In the past, as a technique to freeze an object to be frozen using a freezing medium called brine, a liquid cooling freezing method for freezing an object to be frozen using a freezing medium of, for example, from -20°C to -35°C is known. More specifically, as an example of a liquid cooling freezing method, a technique in which a brine bag with a freezing medium (brine) sealed therein is used and an object to be frozen is sandwiched between brine bags to freeze the object to be frozen is known (see, for example, Patent Document 1).

### CITATION LIST

### Patent Literature

PTL 1: JP2005192533A
PTL 2: JP S6369976 U which discloses a freezing container capable of allowing foods to be quickly frozen with high quality without unevenness irrespective of shapes of food.
PTL 3: JP 3151617 U which discloses a rapid freezing device for food.

### SUMMARY OF INVENTION

### (Technical Problem)

According to the above-described freezing technique using a freezing medium, an object to be frozen can be frozen by using a freezing medium having thermal conductivity higher than that of gas. Thus, different from the blast freezing method (air blast freezing method), when an object to be frozen is frozen, the maximum ice crystal generation temperature zone (0°C to -5°C) can be passed in the shortest possible period of time. Therefore, with a freezing technique using a freezing medium, an object to be frozen can be frozen without causing cell rupture or split (crack). Thus, the above-described liquid cooling freezing method has been preferably used for freezing the food such as meat and fish meat.

In recent years, efficient freezing of an object to be frozen is required in various fields other than the food field. More specifically, in the medical field, for example, efficient freezing of various objects to be frozen such as blood, skin cells, iPS cells, etc. is required. Then, as a technique to freeze such an object to be frozen, a freezing technique using a freezing medium is drawing attention.

However, in the past, the above-described freezing apparatus using a freezing medium has been developed for the purpose of freezing mainly food. Thus, when there is growing diversity of kinds of object to be frozen along with its use in other fields such as a medical field, in some cases, an object to be frozen cannot be frozen efficiently with a conventional freezing apparatus. Specifically, in the medical field, for example, a relatively large object to be frozen such as a transfusion blood pack (whose size is from a few cm to tens of cm) and a very small sample (whose size is about a few mm) such as skin cell or iPS cell samples are needed to be frozen under controlled temperature conditions. However, with the conventional freezing apparatus, it was not possible to freeze objects to be frozen varying in material or shape efficiently within one freezing apparatus.

Therefore the object of the present invention is to provide, when freezing an object to be frozen using a freezing medium, a freezing apparatus capable of efficiently freezing the object to be frozen depending on the material or the shape of the object to be frozen.

### (Solution to Problem)

The present invention is made to solve the above problem. The freezing apparatus according to the present invention is
a freezing apparatus that freezes an object to be frozen using a freezing medium, and has
a first freezing space and a second freezing space whose inner temperatures can be adjusted, wherein
- the first freezing space contains a plurality of freezing medium bags with the freezing medium sealed therein,
- the second freezing space houses the freezing medium such a manner that it can immerse the object to be frozen,
- the freezing apparatus comprises a first drawer forming the first freezing space and a second drawer forming the second freezing space, and
- the second drawer is filled with the freezing medium, and the second drawer has a liquid leakage prevention mechanism that prevents the freezing medium from overflowing to a front side of a drawing direction when the second drawer is drawn.

According to the freezing apparatus, an object to be frozen can be frozen by properly distinguishing between the method of freezing an object to be frozen by sandwiching it between freezing medium bags or the method of freezing the object to be frozen by immersing it directly or by sealing it in a bag, etc. in the freezing medium. Thus the object to be frozen can be frozen efficiently depending on the material or the shape of the object to be frozen. In addition, since the temperature in the freezing space can be adjusted, the object to be frozen can be frozen by using a freezing medium under controlled temperature conditions.

In the freezing apparatus according to the present invention, a first drawer forming the first freezing space and a second drawer forming the second freezing space are provided, thereby facilitating introduction and removal of the object to be frozen to and from the first and the second freezing spaces respectively. In addition, during introduction or removal of the object to be frozen, rise in temperature of the freezing space where introduction and removal of the object to be frozen is not performed can be suppressed.

Furthermore, in the freezing apparatus according to the present invention, it is preferred that, in the second freezing space, the freezing medium is housed in a state where it is contained in a container with a lid, thereby preventing the freezing medium from flowing out from the freezing apparatus accidentally and eliminating the need for filling the whole second freezing space with the freezing medium. Thus the amount of freezing medium to be used can be reduced. In addition, the container with a lid containing the freezing medium that has been cooled to a desired temperature in the freezing apparatus is taken out from the second freezing space, and the lid is opened to immerse the object to be frozen, thereby allowing for a freezing work of the object to be frozen outside the freezing apparatus. Therefore, rise in the temperature in the second freezing space during immersion or removal of the object to be frozen can be suppressed.

Moreover, in the freezing apparatus according to the present invention,
the second drawer is filled with the freezing medium, and the second drawer is provided with a liquid leakage prevention mechanism that prevents the freezing medium from overflowing to a front side of a drawing direction during drawing of the second drawer, thereby, even if the second drawer is directly filled with the freezing medium, preventing the freezing medium from overflowing to the side of an operator (people who draws the drawer) due to the force generated when the drawer is drawn, and enhancing the safety of work.

Furthermore, in the freezing apparatus according to the present invention,
the first freezing space and the second freezing space are disposed vertically adjacent to each other, and
it is preferred that the first freezing space is located on the upper side of the second freezing space, thereby allowing for cooling of the second freezing space effectively by the cold air from the first freezing space side, and for stabilization of the temperature in the second freezing space. In addition, the second freezing space is located on the lower side of the first freezing space, thereby reducing the possibility of attaching the freezing medium to the operator when the freezing medium flows out from the second freezing space, and enhancing the safety of work.

### (Advantageous Effect of Invention)

According to the present invention, a freezing apparatus capable of, when the object to be frozen is frozen using a freezing medium, freezing an object to be frozen efficiently depending on the material or the shape of the object to be frozen can be provided.

### BRIEF DESCRIPTION OF DRAWINGS

The present invention will be further described below with reference to the accompanying drawings, wherein:
FIG. 1A is a perspective view illustrating a freezing apparatus according to one embodiment of the present invention;
FIG. 1B is a drawing illustrating inside of a freezing space of the freezing apparatus illustrated in FIG. 1A; and
FIG. 2 is a perspective view illustrating a freezing apparatus according to another embodiment of the present invention.

### DESCRIPTION OF EMBODIMENTS

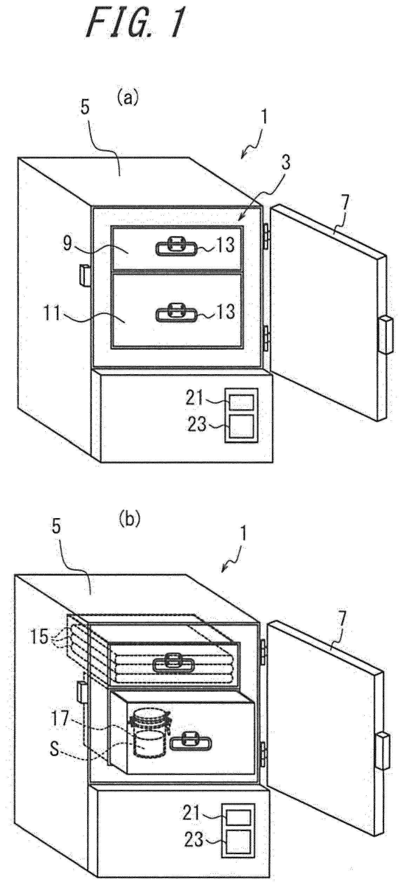

The embodiment of the present invention is exemplarily illustrated below with reference to the drawing. The freezing apparatus 1 illustrated in FIG. 1A has a housing 5 with an opening 3 on the front side thereof and a single swing door 7, which is provided openably/closably on the housing 5 and closes the opening 3 while it is closed. It should be noted that the shape of the housing 5 and of the door 7 and the position of the opening 3 are not particularly limited. In addition, in view of keeping the freezing space inside the freezing apparatus 1 at a desired temperature, it is preferred that the housing 5 and the door 7 have insulating properties.

The freezing apparatus 1 has, inside the housing 5 thereof, a first drawer 9 forming a first freezing space and a second drawer 11 forming a second freezing space. It should be noted that, in this example, although the first freezing space and the second freezing space are disposed vertically adjacent to each other, they may be disposed horizontally adjacent to each other. In addition, the number of the freezing space is not limited to two, and a freezing space in an appropriate form may be provided additionally. In addition, the form of the first and the second freezing spaces is not limited to a drawer, and may be, for example, a form in which the housing 5 is divided like a shelf by using partition members or the like and the side of the opening 3 can be closed by the door 7.

The freezing apparatus 1 has a cooling means (not illustrated) such as, for example, a refrigerant pipe or the like which is disposed outside of each drawer in the housing 5 and through which a refrigerant of a predetermined temperature passes. With this cooling means, the first drawer 9 and the second drawer 11 are cooled respectively and the temperature in each drawer can be adjusted to a desired temperature. It should be noted that the temperature adjustment operation is not particularly limited, and for example, a temperature may be adjusted by changing the temperature and the flow rate of the flowing refrigerant by operating the operation unit 23 comprising buttons, a touch panel, or the like to input a desired setting temperature while confirming the setting temperature and the current inner temperature or the like displayed on the display unit 21 illustrated in FIG. 1A. It should be noted that the temperature in the first drawer 9 and the temperature in the second drawer 11 may be controlled to obtain the same temperature or the different temperature. In addition, the temperatures in the first and the second drawers 9 and 11 are preferably below 0°C, however, in view of freezing of the object to be frozen at an appropriate velocity, the temperatures are preferably below -45°C, and more preferably, below -60°C.

The first drawer 9 and the second drawer 11 can be, with the door 7 opened, drawn by pulling a knob 13 provided on each drawer forward.

The first drawer 9 contains a plurality of freezing medium bags 15 with a freezing medium S sealed therein. In this example, in the first drawer 9, the freezing medium bags 15 are disposed (contained) in three stages. The freezing medium bags 15 and the freezing medium S inside the freezing medium bags are contained in the first drawer 9, and the temperature thereof gradually approaches the temperature in the first drawer 9 and after reaching the temperature, the temperature is kept.

The freezing medium bag 15 is, for example, a plastic bag or the like with the freezing medium S sealed therein. It is preferred that the freezing medium bag 15 has an appropriate flexibility in a degree such that it can change in shape following the outer shape of the object to be frozen and a high thermal conductivity. In addition, the size of the freezing medium bag 15 is not limited to those having almost the same width as that of the bottom face inside the first drawer 9 illustrated in the drawing, and may be any size. As the freezing medium bag 15, a brine bag disclosed in, for example, JP2005192533 (A), may be used.

In the second drawer 11, the freezing medium S is housed such a manner that it can immerse the object to be frozen. In this example, in the second drawer 11, the freezing medium S is housed in a state where it is contained in a container with a lid 17. The container with a lid 17 and the freezing medium S inside the container with a lid 17 are housed in the second drawer 11, the temperature thereof gradually approaches the temperature in the second drawer 11 and after reaching the temperature, the temperature is kept. It should be noted that the shape of the container with a lid 17 is not particularly limited, and may be a cylindrical shape as illustrated. In addition, the container with a lid 17 may preferably be made from metal with an excellent thermal conductivity, and stainless steel suitable for use under low temperature conditions is more preferred.

Although the object to be frozen is not particularly limited, it includes, for example, food such as meat and fish meat, organs, tissues, blood, cultured cells, cultured tissues, blood products or the like of animals including human.

The freezing medium S is not particularly limited unless it is liquid in the operating temperature range, and can be, for example, ethyl alcohol or methyl-butane.

When the freezing apparatus 1 having the above-described configuration is used, the freezing medium bag 15 and the container with a lid 17 are respectively left previously in the first drawer 9 and the second drawer 11 having temperatures adjusted respectively to a desired temperature for a given period of time, and are cooled until they respectively reach the desired temperatures. Then, when the object to be frozen is frozen, either the method in which the object to be frozen is sandwiched between the freezing medium bags 15 contained in the first drawer 9 or the method in which the object to be frozen is immersed in the freezing medium S housed in the second drawer 11 is chosen based on the material, the shape or the like of the object to be frozen. For example, when the specific surface area of the object to be frozen is large, the method in which the object to be frozen is sandwiched between the freezing medium bags 15 in the first drawer 9 may be chosen, and on the other hand, when the specific surface area is relatively small, the method in which the container with a lid 17 is taken out from the second drawer 11 and the lid is opened to immerse the object to be frozen in the freezing medium S, and after the object to be frozen is frozen, the container with a lid 17 is put back into the drawer 11 may be chosen. In addition, other criteria for choosing a freezing means include, for example, when the object to be frozen has a surface free of unevenness and an area in contact with the freezing medium bag 15 is sufficiently ensured, the object to be frozen is sandwiched between the freezing medium bags 15, and on the other hand, when the object to be frozen has an uneven surface, it is immersed in the freezing medium S. Furthermore, another criterion for choosing a freezing means includes, when the object to be frozen is thin and large such as a blood transfusion pack, the object to be frozen is sandwiched between the freezing medium bags 15, and when the object to be frozen is a small preservation container or a cultivation container containing various cell samples such as iPS cells, it is immersed in the freezing medium S.

Therefore, according to the freezing apparatus 1 of the present embodiment, a freezing means is chosen based on the material or the shape of each object to be frozen, thereby allowing for an efficient freezing of the object to be frozen using the freezing medium S. In addition, the method is a freezing method by liquid surface contact using the freezing medium S, which is liquid, thus the object to be frozen is frozen more quickly than the freezing method of gas contact such as general freezers. Thus the cell destruction of the object to be frozen can be suppressed. In addition, the temperature in the freezing space can be adjusted, thereby allowing for freezing of the object to be frozen using the freezing medium S under controlled temperature conditions. Furthermore, the temperature of the freezing medium S actually in contact with the object to be frozen can be obtained, thereby allowing for a study of temperatures suitable for freezing cells, for example.

It should be noted that, in the freezing apparatus 1 of the present embodiment, the first and the second freezing spaces are provided as the drawers 9 and 11 respectively, thereby facilitating introduction and removal of the object to be frozen. In addition, even if the door 7 is left opened, the freezing space can be closed unless the drawer 9 or 11 is drawn, thus rise in the temperature in the freezing space can be suppressed.

Moreover, in the freezing apparatus 1 of the present embodiment, inside the second drawer 11 forming the second freezing space, the freezing medium S is housed in a state where it is contained in the container with a lid 17, thereby preventing the freezing medium S from flowing out accidentally when the second drawer 11 is drawn, and since it is not required to fill the whole second freezing space with the freezing medium S, the amount of the freezing medium S to be used can be reduced. In addition, a freezing work of the object to be frozen can be performed with the container with a lid 17 taken out from the second drawer 11 forming the second freezing space. Thus, rise in the temperature in the freezing space can be suppressed by closing the second drawer 11 and the door 7 during the work.

Furthermore, in the freezing apparatus 1 of the present embodiment, the first drawer 9 forming the first freezing space and the second drawer 11 forming the second freezing space are disposed vertically adjacent to each other, and the first freezing space is located on the upper side of the second freezing space. Thus, cold air from the side of the first drawer 9 containing tightly the freezing medium bags flows into the second drawer 11, thereby allowing for stabilization of the temperature in the freezing space. In addition, the second drawer 11 is located under, thus the container with a lid 17 can be taken out easily and, even when the freezing medium S flows out from the container with a lid 17, the possibility of attaching the freezing medium S to the operator can be reduced and the safety can be enhanced.

Here, when the object to be frozen is blood, the temperature of the freezing medium S is preferably from -80°C to -50°C. With these temperatures, cell destruction may not occur and more reliable freezing at an appropriate velocity can be realized.

In addition, when the object to be frozen is skin tissue and is frozen by immersing in the freezing medium S, the temperature of the freezing medium S is preferably from -50°C to -30°C. With these temperatures, cell destruction may not occur and more reliable freezing at an appropriate velocity can be realized. In the same aspect, when the object to be frozen is frozen by sandwiching between the freezing medium bags 15, the temperature of the freezing medium S in the freezing medium bag 15 is preferably -80°C.

Furthermore, in particular, when a thick object to be frozen is frozen at extremely low temperatures, cracks may occur in the object to be frozen. The reason is that the object to be frozen swells as the freezing proceeds, and heat is generated inside thereof, while the surface thereof is cooled quickly and the temperature thereof goes down. Thus trapped heat may cause cracks. It is preferable that, in order to prevent such cracks, the temperature of the freezing medium S is adjusted according to the thickness of the object to be frozen. The preferable temperature may vary according to the component of the object to be frozen and, for example, when the thickness of the object to be frozen exceeds 1cm, the temperature of the freezing medium S may be set to -40°C or more.

Here, when the object to be frozen is, for example, an object with a complex shape such as a blood pack provided therein with a communicating tube, it is preferred that the whole blood pack is put in a bag or the like and the pack is frozen with the air between the blood pack and the bag removed (deaeration). Thus, even if the object to be frozen has a complex shape, the surface area in contact with the freezing medium is secured, and the object to be frozen can be frozen easily and safely.

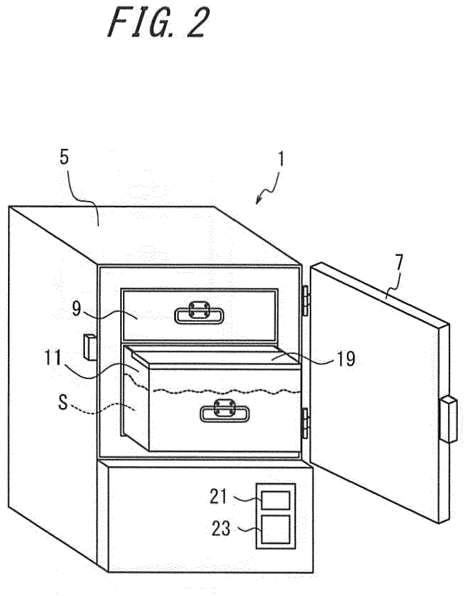

It should be noted that, in the above-described embodiment, an example where, in the second drawer 11, the freezing medium S is housed in a state where it is contained in a container with a lid 17, is illustrated. However, the freezing medium S may be directly filled in the second drawer 11. In this case, for example, as illustrated in FIG. 2, a plate-like lid 19 as a liquid leakage prevention mechanism is provided over the upper portion of the front side of the drawing direction of the second drawer 11, thereby preventing the freezing medium S from overflowing to the front side of the drawing direction when drawing the second drawer 11.

### INDUSTRIAL APPLICABILITY

Thus, according to the present invention, a freezing apparatus capable of freezing the object to be frozen efficiently according to the material or the shape of the object to be frozen, during freezing the object to be frozen using the freezing medium, can be provided.

### REFERENCE SIGNS LIST

- 1: Freezing apparatus
- 3: Opening
- 5: Housing
- 7: Door
- 9: First drawer (first freezing space)
- 11: Second drawer (second freezing space)
- 13: Knob
- 15: Freezing medium bag
- 17: Container with a lid
- 19: Lid (liquid leakage prevention mechanism)
- S: Freezing medium

## Claims

1. A freezing apparatus (1) that freezes an object to be frozen using a freezing medium (S), comprising a first freezing space (9) and a second freezing space (11) whose inner temperatures can be adjusted,
wherein the first freezing space (9) contains a plurality of freezing medium bags (15) with the freezing medium sealed therein,
wherein in the second freezing space (11), the freezing medium (S) is housed in such a manner that it can immerse the object to be frozen,
**characterized in that** the freezing apparatus (1) comprises a first drawer forming the first freezing space (9) and a second drawer forming the second freezing space (11), and
wherein the second drawer is filled with the freezing medium (S), and the second drawer has a liquid leakage prevention mechanism (19) that prevents the freezing medium (S) from overflowing to a front side of a drawing direction when the second drawer (11) is drawn.

2. The freezing apparatus (1) according to claim 1, wherein in the second freezing space (11), the freezing medium (S) is housed in a state where it is contained in a container with a lid (17).

3. The freezing apparatus (1) according to claim 1 or 2, wherein
the first freezing space (9) and the second freezing space (11) are disposed vertically adjacent to each other, and
the first freezing space (9) is located on the upper side of the second freezing space (11).

## Patentansprüche

1. Tiefkühlvorrichtung (1) zum Tiefkühlen eines einzufrierenden Objektes unter Verwendung eines Tiefkühlmediums (S) mit einem ersten Tiefkühlbereich (9) und einem zweiten Tiefkühlbereich (11), deren Innentemperaturen einstellbar sind,
wobei der erste Tiefkühlbereich (9) eine Vielzahl von Tiefkühlmedium-Taschen (15) mit darin eingeschlossenem Tiefkühlmedium beinhaltet,
wobei in dem zweiten Tiefkühlbereich (11) das Tiefkühlmedium (S) in der Weise aufgenommen ist, dass das einzufrierende Objekt darin eintauchbar ist,
**dadurch gekennzeichnet,**
**dass** die Tiefkühlvorrichtung (1) eine erste Schublade, welche den ersten Tiefkühlbereich (9) bildet, und eine zweite Schublade aufweist, welche den zweiten Tiefkühlbereich (11) bildet, und
**dass** die zweite Schublade mit dem Tiefkühlmedium (S) befüllt ist und die zweite Schublade eine Flüssigkeitsauslauf-Schutzeinrichtung (19) aufweist, welche verhindert, dass das Tiefkühlmedium (S) zu einer Frontseite in Schubrichtung überläuft, wenn die zweite Schublade (11) gezogen wird.

2. Tiefkühlvorrichtung (1) nach Anspruch 1,
wobei in dem zweiten Tiefkühlbereich (11) das Tiefkühlmedium (S) in einem Zustand aufgenommen ist, bei dem es in einem Behälter mit einem Deckel (17) aufgenommen ist.

3. Tiefkühlvorrichtung (1) nach Anspruch 1 oder 2,
wobei der erste Tiefkühlbereich (9) und der zweite Tiefkühlbereich (11) vertikal benachbart zueinander angeordnet sind und
der erste Tiefkühlbereich (9) an der Oberseite des zweiten Tiefkühlbereichs (11) angeordnet ist.

## Revendications

1. Appareil de congélation (1) qui congèle un objet à congeler en utilisant un milieu de congélation (S), comprenant un premier espace de congélation (9) et un deuxième espace de congélation (11) dont les températures intérieures peuvent être ajustées,
dans lequel le premier espace de congélation (9) contient une pluralité de sacs de milieu de congélation (15), le milieu de congélation étant enfermé hermétiquement dans ceux-ci,
dans lequel, dans le deuxième espace de congélation (11), le milieu de congélation (S) est logé d'une manière telle qu'il permet d'immerger l'objet à congeler,
**caractérisé en ce que** l'appareil de congélation (1) comprend un premier tiroir formant le premier espace de congélation (9) et un deuxième tiroir formant le deuxième espace de congélation (11), et
dans lequel le deuxième tiroir est rempli du milieu de congélation (S), et le deuxième tiroir comporte un mécanisme de prévention de fuite de liquide (19) qui empêche le milieu de congélation (S) de déborder vers un côté avant d'une direction de traction lorsque le deuxième tiroir (11) est tiré.

2. Appareil de congélation (1) selon la revendication 1, dans lequel, dans le deuxième espace de congélation (11), le milieu de congélation (S) est logé dans un état dans lequel il est contenu dans un contenant avec un couvercle (17).

3. Appareil de congélation (1) selon la revendication 1 ou 2, dans lequel
le premier espace de congélation (9) et le deuxième espace de congélation (11) sont disposés verticalement adjacent l'un à l'autre, et
le premier espace de congélation (9) est situé du côté supérieur du deuxième espace de congélation (11).
